# EUROPEAN PATENT APPLICATION

(11) **EP 4 806 336 A1**
(43) Date of publication of application: **16.09.2026**
(21) Application number: 25163194.1
(22) Date of filing: 12.03.2025
(51) Int. Cl.: A61B 5/00, A61B 5/02, A61B 5/07, A61B 6/50, A61F 2/07, A61F 2/48, A61F 2/90, A61F 2/915, G16H 50/20, A61B 17/22

(54) **IMPLANTABLE MEDICAL DEVICE**

(71) Applicant: Siemens Healthineers AG, 91301 Forchheim (DE)
(72) Inventor: KRISHNA, M. Mohana, 560100 Bangalore (IN); RAJAGOPAL, Lokesh, 560100 Bangalore (IN); VENKATESAN, Hemmath, 560100 Bangalore (IN)
(74) Representative: HKW Intellectual Property PartG mbB

(57) **Abstract**

The present invention relates to an implantable medical device, e.g. a stent, particularly adapted for restenosis identification. Furthermore, the present invention relates to a system, particularly adapted for restenosis identification, the system including such an implantable medical device and a number of readers being coupeable with the implantable medical device.

An implantable medical device comprises a structure having a cylindrical shape and an inner lumen, and a number of radio transponders, the respective radio transponder being configured to transmit a radio signal having a certain frequency to a receiver, particularly adapted for restenosis identification.

Using the radio transponders, it is advantageously possible to monitor the health of the implantable medical device.

## Description

The present invention relates to an implantable medical device comprising a structure having a cylindrical shape and an inner lumen, e.g. a stent, particularly adapted for restenosis identification. Furthermore, the present invention relates to a system, particularly adapted for restenosis identification, the system including such an implantable medical device and a number of readers being coupeable with the implantable medical device.

After an implantable medical device, e.g. a stent or coronary stent, is placed in a artery, several potential complications may arise after the procedure. These potential complications may include restenosis, i.e., a re-narrowing of the treated artery, and stent thrombosis, i.e., the formation of a blood clot within the stent.

To identify the above issues, an invasive diagnostic angiogram is conventionally required. This may result in high radiation redoing the entire procedure.

In particular, in many cases, a repeat of the complete angiography procedures, including ECG, echocardiography, computed tomography, and/or angiography may be needed to re-assess the situation. In large cases of stent restenosis, the patient needs to undergo an intravascular ultrasound or optical coherence tomography (OCT). Disadvantageously, there is no conventional solution available without a radiation exposure for the patient.

Document US9188558B2 discloses a method and a system of monitoring environmental exposure of stents using radiofrequency identification. The disclosed method of monitoring a stent comprises: obtaining readings of an environmental parameter from a sensor adjacent to a stent, the sensor positioned within or on a container including the stent, wherein an RFID tag located within or on the container receives the readings, and wherein the RFID tag and the sensor are integrated as one unit or the RFID tag and the sensor are separate units, transmitting the readings from the RFID tag to a transceiver, wherein a maximum tolerance of the stent for the environmental parameter is stored on the RFID tag, and comparing the readings to the maximum tolerance for the environmental parameter, wherein the environmental parameter is selected from the group consisting of vibration, and shock.

Therefore, it is one object of the present invention to provide an improved implantable medical device.

According to a first aspect, an implantable medical device is suggested, wherein the implantable medical device includes a structure having a cylindrical shape and an inner lumen, and a number N1 of radio transponders, with N1 ≥ 1, the respective radio transponder being configured to transmit a radio signal having a certain frequency to a receiver, particularly adapted for restenosis identification.

For example, the implantable medical device is a stent, and the radio transponder is an RFID tag. In particular, stenosis refers to a narrowing or constriction of the diameter of a passage, e.g. a bodily passage or orifice. Further, restenosis may refer to the reoccurrence of stenosis in the passage, e.g. a blood vessel or heart valve after it has been treated (as by balloon angioplasty, stenting, or valvuloplasty).

By use of the N1 radio transponders, it is advantageously possible to monitor the health of the implantable medical device, in particular without the use of any radiation. Certain particles, like plague, inside and/or outside the implantable medical device may change characteristics of the radio signal transmitted by the radio transponder. Any change of the characteristics of the transmitted radio signal may be detectable by the receiver. Thus, such a change of the characteristics may indicate a change of the health of the implantable medical device.

To monitor the health of the implantable medical device, information describing the particles, e.g. plague formation, can be measured using said reader. In embodiments, this reader can be a hand-held device or part of an angiography system. The reader is adapted to measure the implantable medical device's health, e.g. plague formation, by detecting interference from the plague before and during follow-up check-ups. For example, the percentage of signal attenuation from a radio transponder placed on the implantable medical device can predict restenosis formation.

Using the present implantable medical device provides the advantage that no check angiogram is required during follow-ups for the patient. Furthermore, there is no need for contrast material injection into the veins for the identification of a blockage. Moreover, to verify the status of the implantable medical device, no additional X-ray or fluoroscopy is needed. For example, the present implantable medical device may be used in any stenting procedures, including coronary/neurovascular and peripheral stenting procedures.

According to an embodiment, the structure comprises a number of structural elements defining the cylindrical shape and the inner lumen. In particular, the structure comprises a pattern with a number of interconnecting struts defining the cylindrical shape and the inner lumen.

According to a further embodiment, the implantable medical device is an endoprosthesis. The endoprosthesis is a medical device that may be implanted inside the body of a patient to replace a damaged or missing part of a joint, bone, or other tissue. It may be used to restore function, relieve pain, or improve the quality of life for patients with conditions like arthritis, fractures, or joint degeneration, for example. The endoprosthesis can be made from materials like metal, plastic, or ceramic, and its design depends on the specific body part they are replacing.

According to a further embodiment, the implantable medical device is a stent. A stent is an example of such an endoprosthesis. A stent is a cylindrically shaped device, which functions to hold open and sometimes expand a segment of a vessel, e.g. a blood vessel, or other anatomical lumen such as urinary tracts and bile ducts. A stent may be used in the treatment of atherosclerotic stenosis in blood vessels, for example. A stent, like a coronary stent for example, may have a diameter in the range from 2.5 mm to 4.5 mm and a length in the range of 12 mm to 48 mm. Such a stent with these dimensions may include multiple radio transponders.

According to a further embodiment, the implantable medical device comprises a plurality N1 of radio transponders, with N1≥2. Multiple radio transponders may be adapted to capture the data in a plurality of different directions (or angles) relative to the implantable medical device for calculating accurate and in-depth data on the implantable medical device, exemplary including stent restenosis, type of plague formation, percentage of plague and/or plague formation.

Using different angles may help determining the frequency range of each antenna, considering the data rate, read range, interference range, and/or attenuation at different intervals. In particular, the use of different angles may provide information if there is an excel blockage in between the implantable medical device after the procedure and post-checks.

According to a further embodiment, the N1 radio transponders are configured to transmit radio signals with N2 different frequencies, with 2≤N2≤N1. Using multiple radio transponders with multiple frequencies provides an improvement in calculating accurate and in-depth data on the implantable medical device. For example, if the implantable medical device includes five radio transponders, the five different frequencies may be 125 kHz, 128 kHz, 130 kHz, 12,6 MHz, and 13,53 MHz.

According to a further embodiment, the N1 radio transponders are configured to transmit radio signals with N1 different frequencies, wherein each of the N1 different frequencies is bijectively allocated to one of the N1 radio transponders. In other words, each of the plurality of radio transponders has its own frequency. As the allocation of radio transponders to frequencies is bijective, each one of the plurality of radio transponders corresponds to exactly one frequency, and vice versa.

According to a further embodiment, each of the N2 frequencies is selected from a frequency band between 100 kHz and 100 MHz. In embodiments, different frequencies or frequency bands are possible according to certain applications.

According to a further embodiment, the N1 radio transponders are configured to transmit radio signals with a common frequency. In some applications, it may be beneficial to use a plurality of radio transponders all using a common frequency, i.e. the same frequency, like 125 kHz.

According to a further embodiment, the radio transponder is an RFID tag. In particular, the RFID tag includes a substrate, an antenna, and a chip (or microchip). In particular, the RFID tag consists of the microchip, the substrate and the antenna being placed on said substrate. For example, the microchip of the RFID tag has a diameter of 1.4 mm and a length of 8.5 mm. For example, a coronary stent ranging from 2.5 mm to 4.5 mm in diameter, 12 mm to 48 mm length can accommodate multiple RFID tags. For example, the RFID chip antenna and the substrate may be built into the stent. In particular, the antenna and the substrate may be placed as part of the stent mesh and the chip of the RFID tag may be fixed in any side of the stent. For example, the RFID chip may have an area of 0.15 x 0.15 mm and a thickness of 7.5 µm. Such a chip is disclosed in reference [1], for example.

For the case that the radio transponders are embodied as RFID tags, the reader is embodied as an RFID reader. In particular, the maximum range of an RFID reader is 1.5 m without distraction. But once the present stent including at least one RFID tag is implanted, the reading capacity may vary and can be read effectively with an RFID reader placed close to the patient's body. It may be noted that the lifetime of a passive RFID tag can easily last over 20 years.

In particular, an RFID tag can store information, for example from 64 bits to 1 kB, in particular as the need to store customer data. With encryption, this data may be protected for medical needs so that the RFID tag information may not be exposed to any readers in the vicinity of the implantable medical device. As mentioned above, the RFID tags may use multiple frequencies, and further, the multiple RFID tags may be read from RFID readers arranged in different angles relative to the implantable medical device. Using these means, it is possible to provide accurate and in-depth data on stent restenosis, to accurately calculate a type of plague information (soft or hard plague), the percentage of plague, and/or extra plague in and around the stent area.

According to a further embodiment, the implantable medical device is embodied as a stent and the structure is embodied as a stent mesh. In particular, the substrate and the antenna of each of the N1 RFID tags are placed as part of the stent mesh or are placed in the inner lumen of the stent mesh.

According to a further embodiment, the respective RFID tag is embodied as a passive RFID tag. Advantageously, a passive RFID tag does not emit any radio frequency by default. A passive RFID tag installed inside a stent receives the radio frequency from the RFID readers and emit it. Particularly low frequencies (LF; low frequency) in a range between 128 kHz to 138 kHz may be used (see reference [2]). Particularly, the RFID tag antennas may be covered with polytetrafluoroethylene, which prevents restenosis. These RFID tags can be made liquid-proof with IPX8 rating to avoid tag failure post implant (see references [3] and [4]).

Any embodiment of the first aspect may be combined with any embodiment of the first aspect to obtain another embodiment of the first aspect.

According to a second aspect, a system, particularly adapted for restenosis identification, is suggested. The system comprises an implantable medical device according to the first aspect or to one of the embodiments of the first aspect, and a number N3 of readers, with N3≥1. Each of said N3 readers is configured to receive radio signals transmitted by the N1 radio transponders of the implantable medical device.

According to an embodiment, the system comprises a plurality N3 of readers, with N3≥2. In particular, the N3 readers are located in different angles relative to the implantable medical device. As mentioned above, with this arrangement of positioning a plurality of readers in different angles relative to the implantable medical device, it is possible to accurately calculate in-depth data on stent restenosis, type of plague formation, percentage of plague and/or extra plague formation.

According to a further embodiment, each of said N3 readers is configured to determine signal data based on the received radio signals transmitted by the implantable medical device. In particular, the signal data includes a signal strength, a signal attenuation, and/or a signal interference. By use of the detected signal strength, the detected signal attenuation, and/or the detected signal interior interference, one can derive a stent restenosis, type of plague formation, percentage of plague and/or extra plague formation.

According to a further embodiment, the system further includes a calculation unit which is configured to determine a restenosis information based on the signal data determined by the N3 readers. In particular, the restenosis information includes a first indicator indicating a percentage of plaque allocated to the implantable medical device, a second indicator indicating a type of the plaque allocated to the implantable medical device, and/or a third indicator indicating a plaque formation of the plaque.

The calculation unit may be implemented in hardware and/or in software. If said calculation unit is implemented in hardware, it may be embodied as a device, e.g. as a computer or as a processor or as a part of a system, e.g. a computer system. If said calculation unit is implemented in software, it may be embodied as a computer program product, as a function, as a routine, as a program code or as an executable object.

According to a further embodiment, at least one of the N3 readers is embodied as a separate hand-held device. For example, the hand-held device is an RFID reader.

According to a further embodiment, at least one of the N3 readers is embodied as part of an angiography system. In particular, the angiography system is an angiography solution for interventional radiology. For example, the angiography system is an ARTIS angiosystem.

The embodiments and features described with reference to the implantable medical device apply mutatis mutandis to the system.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematic illustration of a first embodiment of an implantable medical device;
Fig. 2 shows a further view of the first embodiment of the implantable medical device according to Fig. 1;
Fig. 3 shows a schematic illustration of a second embodiment of an implantable medical device having a plurality of radio transponders;
Fig. 4 shows a schematic illustration of a third embodiment of an implantable medical device having a plurality of radio transponders being implanted in a vessel;
Fig. 5 shows a further view of the third embodiment of Fig. 4 illustrating stenosis identification using a multi direction detection;
Fig. 6 shows a schematic illustration of a first embodiment of a system adapted for restenosis identification; and
Fig. 7 shows a schematic illustration of a second embodiment of a system adapted for restenosis identification.

In the Figures, like reference numerals designate like or functionally equivalent elements, unless otherwise indicated.

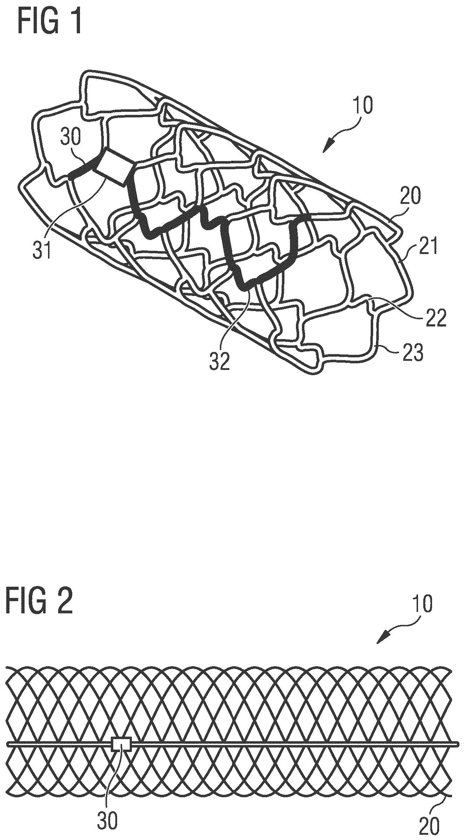

In Fig. 1, a schematic illustration of a first embodiment of an implantable medical device 10 is depicted. Particularly, the implantable medical device 10 is an endoprosthesis, for example a stent. In this regard, Fig. 2 shows a further view of the first embodiment of the implantable medical device 10 according to Fig. 1.

The implantable medical device of Figs. 1 and 2 comprises a structure 20 having a cylindrical shape and an inner lumen. In particular, the structure 20 has a number of structural elements defining the cylindrical shape and the inner lumen. With respect to Fig. 1, only three of said structural elements 21-23 are comprised with reference signs to increase the readability of Fig. 1. As Fig. 1 further shows, the structure 20 may be embodied as a pattern having a number of interconnecting struts 21-23 defining the cylindrical shape and the inner lumen. For the case that the implantable medical device is embodied as a stent, as shown in Figs. 1 and 2, the structure may be embodied as a stent mesh.

Furthermore, with reference to Figs. 1 and 2, the implantable medical device 10 includes a number N of radio transponders 30, with N1≥1. Without loss of generality, the embodiment of Figs. 1 and 2 has one radio transponder 30, with N1=1. The radio transponder 30 may be embodied as an RFID tag. The RFID tag 30 includes a substrate, an antenna 32 which is located on said substrate and a chip 31 (which may be also referred to as microchip or RFID chip).

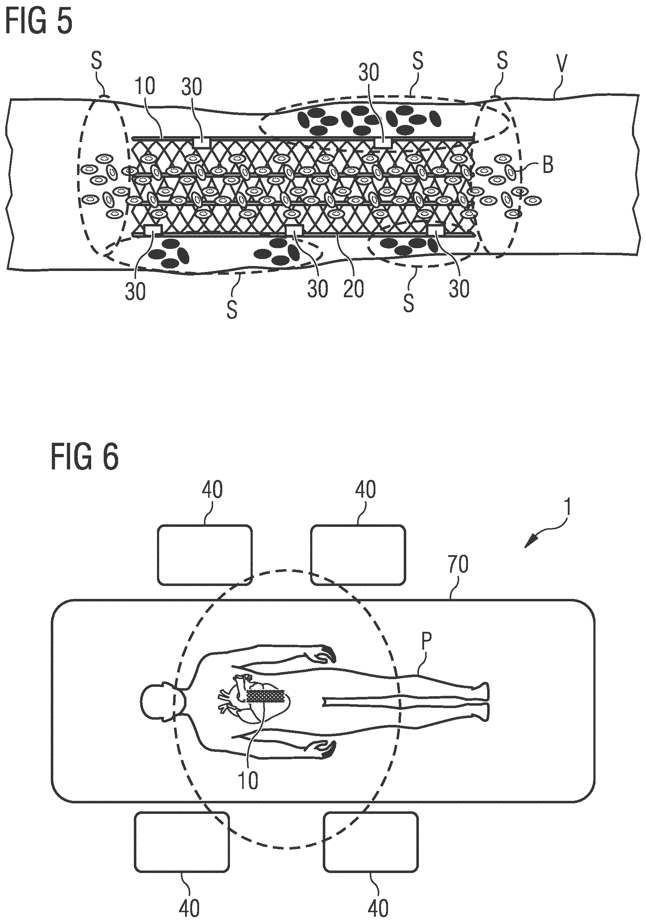

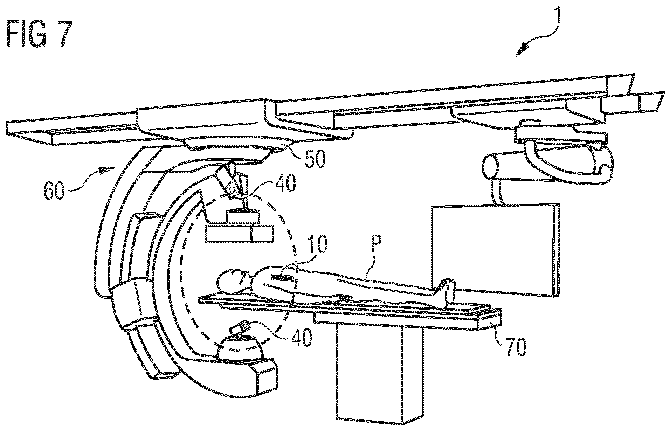

The radio transponder 30, for example embodied as RFID tag, is configured to transmit a radio signal having a certain frequence to a receiver 40 (see Figs. 6 and 7). The transmitted radio signal is particularly adapted for restenosis identification, in particular in a case where the implantable medical device 10 is implanted in a blood vessel of a patient P (see Fig. 7).

For example, the substrate and the antenna 32 of the RFID tag 30 are placed as part of the stent mesh 20 of the stent 10. In alternative embodiments, the substrate and the antenna 32 of the RFID tag 30 are placed in the inner lumen of the stent mesh 20.

By use of the N1 radio transponders 30, it is advantageously possible to monitor the health of the implantable medical device 10, in particular without the use of any radiation. Certain particles, like plague, inside and/or outside the implantable medical device 10 may change characteristics of the radio signal transmitted by the radio transponder 30. Any change of the characteristics of the transmitted radio signal may be detectable by the receiver 40. Thus, such a change of the characteristics may indicate a change of the health of the implantable medical device 10.

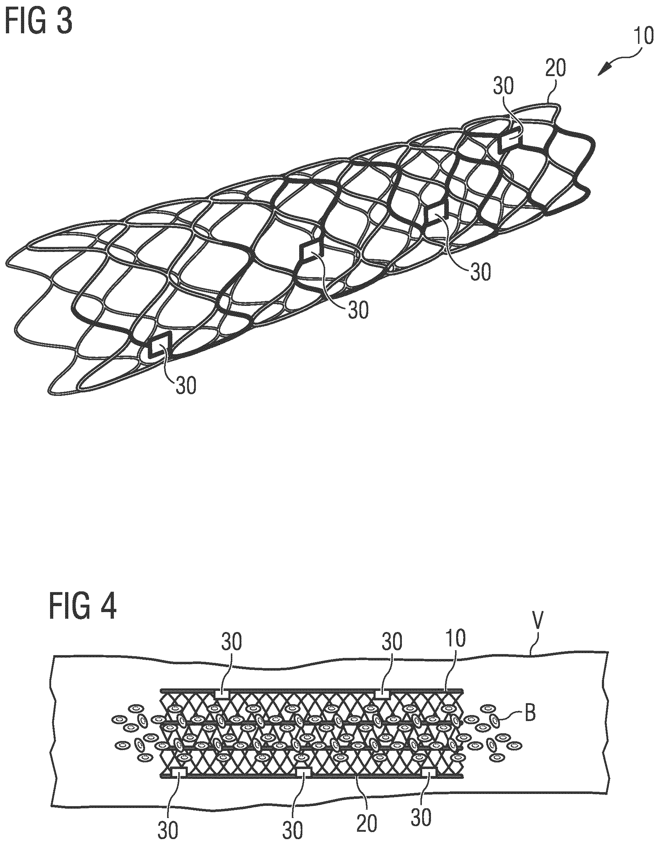

Fig. 3 shows a schematic illustration of a second embodiment of an implantable medical device 10 having a plurality of radio transponders 30. Each of the radio transponders 30 of Fig. 3 may be embodied as an RFID tag. The implantable medical device 10 of Fig. 3 is embodied as a stent. Without loss of generality, the stent 10 of Fig. 3 includes four radio transponders 30 (with N1 = 4), particularly embodied as a respective RFID tag 30. To improve readability, the single parts of the respective RFID tag, i.e. the RFID chip 31 and the antenna 32 with the underlying substrate, are not provided with dedicated reference signs in Fig. 3.

In particular, the N1 RFID tags 30 (with N1=4) of Fig. 3 are configured to transmit radio signals with N2 different frequencies, with 2≤N2≤N1. For example, each of the N2 frequences is selected from a frequency band between 100 kHz and 100 MHz.

In some applications, the N1 radio transponders 30 are configured to transmit radio signals with N1 different frequencies. In these cases, each of the N1 different frequencies is bijectively allocated to one of the N1 radio transponders 30. Multiple radio transponders 30 are configured to capture the data in a plurality of different directions (or angles) relative to the implantable medical device 10 to calculate accurate and in-depth data on the implantable medical device 10. The data may exemplarily include stent restenosis, type of plague formation, percentage of plague and/or plague formation.

In some alternative embodiments, the N1 radio transponders 30 are configured to transmit radio signals with a common frequency, e.g. 150 kHz.

With respect to Fig. 3, multiple RFID tags 30 with different frequencies or an equal frequency can be placed in or on the stent 10. With the help of an RFID reader 40 (see Figs. 6 and 7) either hand-held or integrated into an angiography system 60, one can measure the strength of each RFID tag signal. Using RFID tags 30 with different frequencies may help identifying interference and attenuation due to plague or restenosis.

Furthermore, Fig. 4 shows a schematic illustration of a third embodiment of an implantable medical device 10 having a plurality of radio transponders 30 being implanted in a vessel V. Without loss of generality, the implantable medical device 10 of Fig. 4 is a stent, the radio transponders 30 are embodied as RFID tags, and the number of RFID tags 30 in Fig. 4 is five (N1=5).

If an RFID reader 40 (not shown in Fig. 4) is placed anywhere in the vicinity of the stent 10 of Fig. 4, the RFID reader 40 has a respective different angle relative to each one of the five RFID tags 30. Because of these different angles, a multi-direction detection is possible which provides an improved restenosis identification.

In this regard, Fig. 5 shows a further view of the third embodiment of Fig. 4 illustrating five different exemplary areas S of restenosis outside the stent area of the stent 10.

Using the multiple RFID tags 30 in the stent 10 and, therefore, the different angles to an RFID reader 40 provides that multi-direction detection and therefore the possibility to distinguish stenosis outside the stent area and stenosis inside the stent area.

Further, Fig. 6 shows a schematic illustration of a first embodiment of a system 1 adapted for restenosis identification. In this regard, Fig. 6 shows a patient P lying on a bed 70. In the vicinity of the bed 70, there are located four readers 40, for example embodied as RFID readers 40. Furthermore, Fig. 6 shows that an implantable medical device 10, e.g. a stent, is implanted in a vessel of the patient P laying on said bed 70. The four RFID readers 40 have different angles relative to said stent 10. In particular, said stent 10 has a plurality of radio transponders 30 as discussed with reference to Fig. 3 to 5. Each of said readers 40 is configured to receive radio signals transmitted by the radio transponders 30 of the stent 10.

The RFID readers 40 of Fig. 6 may be embodied as hand-held devices, for example, or may be integrated in an angiography system 60 which is discussed with reference to Fig. 7 in detail.

Thus, each of said readers 40 is configured to determine signal data including a signal strength, a signal attenuation and/or a signal interference based on the received radio signals transmitted by said stent 10. Based on the determined signal data, a restenosis information of said stent 10 may be determined. The restenosis information may include a first indicator indicating a percentage of plague allocated to the stent 10, a second indicator indicating a type of the plague allocated to the stent 10 and a third indicator indicating a plague formation of the plague.

Further, Fig. 7 shows a schematic illustration of a second embodiment of the system 1 adapted for restenosis identification. The system 1 of Fig. 7 includes an angiography system 60. Without loss of generality, the angiography system 60 of Fig. 7 comprises two RFID readers 40 arranged at the C-arm of the angiography system 60. The system 1 of Fig. 7 further includes a bed 70 on which a patient P is lying. A stent 10 is implanted in a vessel of said patient P. Radio signals transmitted by the RFID tags 30 of the stent 10 may be read by the RFID readers 40 of said angiography system 60.

The angiography system 60 may further include a calculating unit 50 which is coupled with said RFID readers 40. The calculation unit 50 may be adapted to determine a restenosis information based on the signal data determined by the RFID readers 40. The RFID readers 40 provide the signal data based on the received radio signals transmitted by the stent 10 of the patient P. The restenosis information provided by said calculation 50 may include above-discussed first indicator, the second indicator and/or the third indicator.

Although the present invention has been described in accordance with preferred embodiments, it is obvious for the person skilled in the art that modifications are possible in all embodiments.

### List of Reference

- 1: system
- 10: implantable medical device, e.g. stent
- 20: structure, e.g. stent mesh
- 21: structural element, e.g. strut
- 22: structural element, e.g. strut
- 23: structural element, e.g. strut
- 30: radio transponder, e.g. RFID tag
- 31: chip, e.g. RFID chip
- 32: antenna with underlying substrate
- 40: receiver, e.g. RFID receiver
- 50: calculation unit
- 60: angiography system
- 70: bed

- B: blood
- P: patient
- S: stenosis
- V: vessel

References
[1] World's smallest and thinnest 0.15 x 0.15 mm, 7.5µm thick RFID IC chip, Enhanced productivity enabled by 1/4 surface area, 1/8th thickness, February 6, 2006 (https://www.hitachi.com/New/cnews/060206.html#:~:text=HITACHI%20GLOBAL% 20%3A%20News%20Release%20%3A%20World's,7.5%C2%B5m%20thick%20RFID %20IC%20chip
[2] https://www.arpansa.gov.au/understanding-radiation/what-is-radiation/non-ionising-radiation/radiofrequency-radiation [3]
[3] https://www.encstore.com/blog/5820-understanding-ip-ratings-in-rfid-and-barcoding-devices
[4] https://www.intechopen.com/chapters/45030

## Claims

1. An implantable medical device (10), comprising
a structure (20) having a cylindrical shape and an inner lumen, and
a number N1 of radio transponders (30), with N1 ≥ 1, the respective radio transponder (30) being configured to transmit a radio signal having a certain frequency to a receiver (40), particularly adapted for restenosis identification.

2. The implantable medical device according to claim 1,
wherein the structure (20) comprises a number of structural elements (21 - 23) defining the cylindrical shape and the inner lumen, particularly, the structure (20) comprising a pattern with a number of interconnecting struts (21 - 23) defining the cylindrical shape and the inner lumen.

3. The implantable medical device according to claim 1 or 2,
wherein the implantable medical device (10) is an endoprosthesis or a stent.

4. The implantable medical device according to one of claims 1 to 3,
wherein the implantable medical device (10) comprises a plurality N1 of radio transponders (30), with N1≥2.

5. The implantable medical device according to claim 4,
wherein the N1 radio transponders (30) are configured to transmit radio signals with N2 different frequencies, with 2≤N2≤N1.

6. The implantable medical device according to claim 5,
wherein the N1 radio transponders (30) are configured to transmit radio signals with N1 different frequencies, wherein each of the N1 different frequencies is bijectively allocated to one of the N1 radio transponders.

7. The implantable medical device according to claim 5 or 6,
wherein each of the N2 frequencies is selected from a frequency band between 100 kHz and 100 MHz.

8. The implantable medical device according to claim 4,
wherein the N1 radio transponders (30) are configured to transmit radio signals with a common frequency.

9. The implantable medical device according to one of claims 1 to 8,
wherein the radio transponder (30) is an RFID tag, the RFID tag (30) including a substrate, an antenna (32), and a chip (31).

10. The implantable medical device according to claim 9,
wherein the implantable medical device (10) is embodied as a stent and the structure (20) is embodied as a stent mesh,
wherein the substrate and the antenna (32) of each of the N1 RFID tags (30) are placed as part of the stent mesh (20) or are placed in the inner lumen of the stent mesh (20).

11. The implantable medical device of claim 9 or 10,
wherein the respective RFID tag (30) is embodied as a passive RFID tag.

12. A system (1), particularly adapted for restenosis identification, the system (1) comprising:
an implantable medical device (10) according to one of claims 1 to 11, and
a number N3 of readers (40), with N3≥1, each of said N3 readers (40) being configured to receive radio signals transmitted by the N1 radio transponders (30) of the implantable medical device (10).

13. The system according to claim 12,
wherein the system (1) comprises a plurality N3 of readers (40), with N3≥2, wherein the N3 readers (40) are located in different angles relative to the implantable medical device (10).

14. The system according to claims 13,
wherein each of said N3 readers (40) is configured to determine signal data including a signal strength, a signal attenuation and/or a signal interference based on the received radio signals transmitted by the implantable medical device (10).

15. The system according to claim 14, further comprising:
a calculation unit (50) which is configured to determine a restenosis information based on the signal data determined by the N3 readers (40), the restenosis information particularly including a first indicator indicating a percentage of plaque allocated to the implantable medical device (10), a second indicator indicating a type of the plaque allocated to the implantable medical device, and/or a third indicator indicating a plaque formation of the plaque.

16. The system according to one of claims 12 to 15,
wherein the respective one of the N3 readers (40) is embodied as a separate hand-held device or as part of an angiography system (60).
